Europäisches Patentamt

European Patent Office

Office européen des brevets

(11) Veröffentlichungsnummer: **0 467 169 B1**

## (12) EUROPÄISCHE PATENTSCHRIFT

(45) Veröffentlichungstag der Patentschrift: **05.10.94**

(21) Anmeldenummer: **91111179.7**

(22) Anmeldetag: **05.07.91**

(51) Int. Cl.⁵: **C07C  45/38**, C07C 47/04

54 **Verfahren zur Herstellung von Formaldehyd.**

(30) Priorität: **16.07.90 DE 4022603**

(43) Veröffentlichungstag der Anmeldung:
**22.01.92 Patentblatt  92/04**

(45) Bekanntmachung des Hinweises auf die
Patenterteilung:
**05.10.94 Patentblatt  94/40**

(84) Benannte Vertragsstaaten:
**BE DE ES FR GB IT NL SE**

(56) Entgegenhaltungen:
**EP-A- 0 271 812**

**CHEMICAL ABSTRACTS, Band 105, 1986, Seite 103, Spalte 1, Zusammenfassung Nr.
210789b, Columbus, Ohio, US; & CN-A-85 100
530**

**PATENT ABSTRACTS OF JAPAN, Band 8, Nr.
41 (C-211)[1478], 22. Februar 1984; & JP-A-58
203 928**

**PATENT ABSTRACTS OF JAPAN, Band 8, Nr.
130 (C-229)[1567], 16. Juni 1984; & JP-A-59 39
843**

(73) Patentinhaber: **BASF Aktiengesellschaft
Carl-Bosch-Strasse 38
D-67063 Ludwigshafen (DE)**

(72) Erfinder: **Aicher, Albrecht
Sonnenstrasse 21
W-6710 Frankenthal (DE)**
Erfinder: **Disteldorf, Walter, Dr.
Portugieser Weg 17
W-6706 Wachenheim (DE)**
Erfinder: **Petri, Norbert, Dr.
Max-Beckmann-Strasse 17
W-6710 Frankenthal (DE)**
Erfinder: **Reuss, Günther, Dr.
Theaterplatz 10
W-6700 Ludwigshafen (DE)**

**Beschreibung**

Die vorliegende Erfindung betrifft ein verbessertes Verfahren zur Herstellung von Formaloenyd durch oxidative Dehydrierung von Methanol an einem Silber enthaltenden, in einem senkrecht stehenden Ronrreaktor angeordneten Festbettkatalysator.

Aus der EP-A-0104.666 ist ein Verfahren zur oxidativen Dehydrierung von Methanol an einem Silberkatalysator bekannt, bei dem das dem Reaktor zugeführte Methanol flüchtige Phosphorverbindungen enthält. Durch den Zusatz der Phosphorverbindungen zum Methanol kann die Selektivität bei der Umsetzung zu Formaldenyd verbessert werden. Nachteile dieses Verfahrens sind darin zu sehen, Gas die Phosphorverbindungen kontinuierlich zudosiert werden müssen, da sie den Reaktor mit dem Produktstrom verlassen. Im Produkt sind daher Phosphorverbindungen nachweisbar.

In der japanischen Offenlegungsschrift JP-A-38 227/83 wird ein Silberkatalysator für die Herstellung von Aldehyden durch Oxidation von Alkoholen beschrieben, der durch Verschmelzen von Silber mit Phosphor oder Imprägnieren des Silberkatalysators mit einer Lösung von Phosphor oder einer Phosphorverbindung, wie Phosphorsäure oder wäßrigen Lösungen von Phosphaten wie Ammnoniumdihydrogenphosphat, erhalten wird. Ein so hergestellter Silberkatalysator wird erst nach einiger Zeit bei hohen Temperaturen aktiv und gibt nach relativ kurzer Zeit Phosphor ab, so daß kein selektivitätsverbessernder Effekt verbleibt.

Aus der chinesischen Patentanmeldung CN-A-85100530 ist ein Silberkatalysator bekannt, der ebenfalls durch Imprägnieren des Silberkatalysators mit einer wäßrigen Lösung von Phosphorsäuren oder beliebigen Natriumphosphaten oder durch Aufdampfen von Phosphor auf den Silberkatalysator erhalten wurde.

Aus der US-A-2 656 322 und der GB-A-1 272 592 sind Silberkatalysatoren bekannt, die Phosphor als Legierungsbestandteil enthalten oder durch Eintauchen von Silber in Orthophosphorsäure mit Phosphor modifiziert worden sind.

In der EP-B-91 060 wird ein aus mehreren Schichten aufgebauter Silberkatalysator beschrieben, bei dem die Silberteilchen der unteren Schicht mit Phosphorsäure behandelt sind.

Nachteile der zuletzt genannten Silberkatalysatoren liegen vor allem auch in der aufwendigen Herstellung und Aufarbeitung der Katalysatoren.

Der Erfindung lag daher die Aufgabe zugrunde, den Nachteilen abzuhelfen, welche mit der Verwendung der bisher bekannten Phosphor enthaltenden Silberkatalysatoren bei der oxidativen Dehydrierung von Methanol zu Formaldehyd verbunden sind.

Demgemäß wurde ein Verfahren zur Herstellung von Formaldehyd durch oxidative Dehydrierung von Methanol an einem Silber enthaltenden, in einem senkrecht stehenden Rohrreaktor angeordneten Festbettkatalysator, dadurch gekennzeichnet, daß man hierzu einen Festbettkatalysator verwendet, der ein pulverförmig aufgebrachtes phosphorhaltiges Salz enthält, dessen Schmelz- oder Zersetzungstemperatur über der Reaktionstemperatur liegt und dessen Menge 0,1 bis 100 mg Phosphor pro $cm^2$ der Festbettkatalysatorquerschnittsfläche beträgt, gefunden.

Für das Verfahren geeignete Ausgangsstoffe sind reines Methanol, technisches Methanol, nach einem Hochdruck- oder Niederdruck-Verfahren hergestelltes Rohmethanol oder vorteilhaft deren Mischungen mit Wasser; die Methanolkonzentration der wäßrigen Gemische beträgt zweckmäßigerweise 60 bis 95 Gew.-%, vorzugsweise 70 und 90 Gew.-%. In einer vorteilhaften Ausführungsform wird Rohmethanol, das nach den in der DE-B-12 77 834, DE-C-12 35 881 und DE-C-11 36 318 beschriebenen Verfahren durch Abtrennung einer niedriger siedenden Fraktion bzw. durch Behandlung mit Oxidationsmitteln und/oder Alkalien gereinigt wurde, verwendet.

Das Methanol wird dem Reaktorraum in Dampfform zugeführt, vorteilhaft im Gemisch mit Nasserdampf und gegebenenfalls mit einem Inertgas. Als Inertgas kommt für das Verfahren beispielsweise Stickstoff in Betracht.

Als oxidierendes Agens lassen sich sowohl reiner Sauerstoff als auch Sauerstoff enthaltende Gase, insbesondere Luft, verwenden. Sauerstoff, und Methanol werden zweckmäßigerweise im Molverhältnis von 0,25 bis 0,6, inbesondere von 0,35 bis 0,5 Mol Sauerstoff pro Mol Methanol verwendet. Vorzugsweise beträgt die Gesamtmenge an Wasserdampf nicht mehr als 3,0, vorteilhaft von 0,67 bis 1,75 mol pro Mol Methanol.

Die Ausgangsstoffe werden durch einen Silber enthaltenden Festbettkatalysator geleitet, welcher in einem senkrecht stehenden Rorreaktor eingebracht ist. Der Katalysator besteht bevorzugt aus Silberkristallen mit einer Korngröße von 0,1 bis 3 mm, insbesondere von 0,2 bis 2,5 mm. Der Festbettkatalysator kann durch Anordnung der Silberkristalle in Schichten mit unterschiedlichen Korngrößen einen Mehrschichtaufbau aufweisen.

Bevorzugt weist die Katalysatorschicht einen Mehrschichtaufbau auf, wie in der DE-B-23 22 757 beschrieben wird. Im unteren Teil der Katalysatorschicht befinden sich bei dem dort beschriebenen Mehrschichtkatalysator 72,5 bis 89, insbesondere 77,5 bis 82.5 Gew.-% aller Katalysatorteilchen, im mittleren Teil 2,5 bis 7,5, insbesondere 4,5 bis 6,5 Gew.-% und im oberen, dem Ausgangsgemisch zugewandten Teil 8,5 bis 20, insbesondere 13 bis 16 Gew.-% aller Katalysatorteilchen. Die Teilchen des unteren Schichtteils haben mittlere Korngrößen von 1 bis 2,5, die des mittleren Schichtteils von 0,75 bis 1 und die des oberen Schichtteils von 0,2 bis 0,75 mm. Die Gesamtschichtdicke des Katalysators beträgt vorzugsweise 5 bis 50 mm.

Das Ausgangsgemisch aus Methanoldampf und Sauerstoff bzw. Luft und gegebenenfalls Wasserdampf und Inertgas wird im Rohrreaktor bevorzugt von oben nach unten geführt, so daß die oberste Schicht dem Ausgangsgemisch zugewandt ist.

Der Festbettkatalysator enthält ein pulverförmiges, phosphorhaltiges Salz, welches eine Schmelz- bzw. Zersetzungstemperatur oberhalb der Reaktionstemperatur im Rohrreaktor aufweist. Bevorzugt ist das phosphorhaltige Salz auf der Seite des Festbettkatalysators aufgebracht, welche dem Ausgangsgemisch zugewandt ist. Besonders bevorzugt ist das Salz oben auf den Festbettkatalysator als Schicht aufgebracht, wenn das Ausgangsgemisch im Rohrreaktor von oben nach unten geführt wird.

Bevorzugt weist das Salz eine Schmelz- oder Zersetzungstemperatur über 800 °C auf. Als Salze kommen z.B. anorganische Phosphate von Alkali- und Erdalkalimetallen, Übergangsmetallen z.B. Ag, Zn, Fe von Bor oder Ammonium in Betracht.

Bevorzugt sind Phosphate oder Pyrophosphate von Alkali- oder Erdalkalimetallen, z.B. $Na_4P_2O_7$, $Li_3PO_4$, $Mg_3(PO_4)_2$, $Ca_3(PO_4)_2$. Die Korngröße der eingesetzten pulverförmigen Salze ist nicht kritisch. So konnte bei handelsüblichen Phospaten unterschiedlicher Korngrößen keine wesentliche Abhängigkeit der katalytischen Wirksamkeit von der Korngröße festgestellt werden. Phosphor enthaltende Salze mit sehr großen Korngrößen, etwa über 1 mm können sich aber ungünstig auf den Strömungsverlauf im Rohrreaktor auswirken, während Salzen mit sehr kleinen Durchmessern, etwa weniger als 1,0 $\mu$m unter Umständen mit der Strömung aus dem Reaktor ausgetragen werden können.

Die Menge des phosphorhaltigen Salzes beträgt 0,1 bis 100 mg Phosphor, pro $cm^2$ der Festbettkatalysatorquerschnittsfläche, vorzugsweise 0,5 bis 50 mg Phosphor (berechnet als Phosphorgehalt im Salz). Zweckmäßigerweise belastet man den Katalysator mit 1 bis 4 t, insbesondere 1,4 bis 2,4 t Methanol pro $m^2$ Katalysatorbettquerschnitt und Stunde. Zur großtechnischen Ausführung verwendet man bevorzugt Katalysatorbettdurchmesser von mindestens 0,5, zweckmäßigerweise 1 bis 3 m. Der Rohrreaktor kann auch zwei oder mehrere, Katalysatorfestbette enthalten.

Das Verfahren wird im übrigen in an sich bekannter Weise durchgeführt, indem man z.B. ein Gasgemisch aus Methanoldampf, Luft, gegebenenfalls Inertgas und zweckmäßig Wasserdampf in vorgenannten Mengen bei Temperaturen von etwa 550 bis 750 °C, insbesondere 600 bis 710 °C, durch das Katalysatorfestbett leitet. Das Verfahren wird im allgemeinen bei einem Druck zwischen 0,5 und 2 bar, vorzugsweise zwischen 0,8 und 1,8 bar, kontinuierlich durchgeführt. Dabei ist es vorteilhaft, die die Katalysatorzone verlassenden Reaktionsgase innerhalb kurzer Zeit abzukühlen, z.B. auf Temperaturen von 50 bis 350 °C. Das abgekühlte Gasgemisch wird dann zweckmäßigerweise einem Absorptionssturm zugeführt, in welchem der Formaldehyd mit Wasser, vorteilhaft im Gegenstrom, aus dem Gasgemisch gewaschen wird.

Eine nennenswerte Austragung des phosphorhaltigen Salzes tritt während der Reaktion nicht auf. Im Produkt konnte kein Phosphor bzw. keine Phosphorverbindungen nachgewiesen werden. Eine wesentliche Verzögerung beim Anspringen der Reaktion ist nicht zu beobachten. Durch das Verfahren wird eine Verbesserung der Ausbeute an Formaldehyd und der Selektivität bezüglich Formaldehyd erreicht.

Die Vorteile des erfindungsgemäßen Verfahren bestehen auch darin, daß der Silberkatalysator nicht in gesonderter Weise mit Phosphor oder Phosphorverbindungen vorbehandelt werden muß. Die phosphorhaltigen Salze können nach Einbringen des Silberkatalysators in den Reaktor einfach auf die oberste Schicht des Katalysators gegeben werden. Auch bei der Aufarbeitung des Silberkatalysators, z.B. durch Elektrolyse können störende Phosphorverunreinigungen vermieden werden, da die Salze durch einfache Trennverfahren z.B. durch Auswaschen mit Wasser vom Silberkatalysator getrennt werden können.

Beispiel

Es wurde ein senkrecht stehender Versuchsreaktor mit einem Innendurchmesser von 15 cm verwendet, in dessen oberen Teil ein Katalysatorfestbett aus drei Schichten von insgesamt 2 cm Höhe angebracht war. Die untere Schicht bestand aus 1000 g Silber der Korngröße von 1 bis 2,5 mm, die mittlere Schicht aus 65 g Silber der Korngröße von 0,75 bis 1 mm und die obere Schicht aus 185 g Silber der Korngröße 0,2 bis 0,75 mm. Auf der oberen Schicht waren pulverförmige Phosphorsalze aufgebracht. Art und Menge dieser Salze ist der nachstehenden Tabelle zu entnehmen.

Pro Stunde wurde ein dampfförmiges Gemisch aus 32 kg Methanol, 21,3 kg Wasser und 45 kg Luft von oben nach unten durch das Katalysatorbett geleitet. Im Anschluß an die Umsetzung wurde das Gemisch auf 150°C abgekühlt und in Wasser aufgenommen.

Tabelle

| Versuchs-Nr. | Temperatur °C | Phosphat | Phosphor mg/cm² | Formaldehyd 100 % kg/h | Rest-methanol bezogen auf Form-aldehyd 100 % | Umsatz an Methanol % | Ausbeute an Formaldehyd % | Selektivität % |
|---|---|---|---|---|---|---|---|---|
| 1* | 700 | – | – | 26,4 | 3,0 | 97,5 | 88,0 | 90,2 |
| 2 | 660 | $Na_4P_2O_7$ | 5,3 | 24,9 | 14,1 | 89,0 | 83,0 | 93,3 |
| 3 | 700 | $Na_4P_2O_7$ | 5,3 | 27,1 | 2,2 | 98,1 | 90,4 | 92,2 |
| 4 | 720 | $Na_4P_2O_7$ | 5,3 | 27,3 | 1,5 | 98,7 | 91,0 | 92,2 |
| 5 | 740 | $K_4P_2O_7$ | 5,3 | 27,2 | 2,0 | 98,3 | 90,5 | 92,0 |
| 6 | 700 | $Li_3PO_4$ | 2,65 | 27 | 3,0 | 97,5 | 90,2 | 92,5 |
| 7 | 720 | $Mg_3(PO_4)_2 \cdot 8\ H_2O$ | 5,3 | 26,9 | 3,0 | 97,4 | 89,6 | 92,0 |
| 8 | 700 | $Ca_3(PO_4)_2$ | 2,65 | 27,2 | 2,7 | 97,7 | 90,7 | 92,8 |
| 9 | 700 | $FePO_4$ | 5,3 | 26,8 | 3,4 | 97,2 | 89,4 | 92,0 |
| 10 | 720 | $Na_4P_2O_7$ | 2,65 | 27,3 | 1,9 | 98,4 | 90,9 | 92,4 |

* zum Vergleich

**Patentansprüche**

1. Verfahren zur Herstellung von Formaldehyd durch oxidative Dehydrierung von Methanol an einem Silber enthaltenden, in einem senkrecht stehenden Rohrreaktor angeordneten Festbettkatalysator, dadurch gekennzeichnet, daß man hierzu einen Festbettkatalysator verwendet, der ein pulverförmig aufgebrachtes phosphorhaltiges Salz enthält, dessen Schmelz- oder Zersetzungstemperatur über der Reaktionstemperatur liegt und dessen Menge 0,1 bis 100 mg Phosphor pro $cm^2$ der Festbettkatalysatorquerschnittsfläche beträgt.

**Claims**

1. A process for the preparation of formaldehyde by oxidative dehydrogenation of methanol over a silver-containing fixed-bed catalyst arranged in a vertical tube reactor, wherein a fixed-bed catalyst which contains a phosphorus-containing salt which is applied in powder form, whose melting point or decomposition temperature is above the reaction temperature and the amount of which corresponds to 0.1-100 mg of phosphorus per $cm^2$ of the cross-sectional area of the fixed-bed catalyst, is used for this purpose.

**Revendications**

1. Procédé de fabrication du formaldéhyde par la déshydratation oxydante du méthanol sur un catalyseur à lit fixe, contenant de l'argent, disposé dans un réacteur tubulaire vertical, caractérisé en ce que l'on utilise, à cette fin, un catalyseur à lit fixe qui contient un sel phosphoré, appliqué sous forme pulvérulente, dont la température de fusion ou de décomposition est supérieure à la température réactionnelle et dont la quantité varie de 0,1 à 100 mg de phosphore par $cm^2$ de surface transversale du catalyseur à lit fixe.